# EUROPEAN PATENT APPLICATION

(11) **EP 4 393 554 A1**
(43) Date of publication of application: **03.07.2024**
(21) Application number: 23211154.2
(22) Date of filing: 21.11.2023
(51) Int. Cl.: A62B 35/00, A41D 13/05, A61F 5/02

(54) **WAIST PADDING ASSEMBLY**

(30) Priority: 28.12.2022 CN 202211691994
(71) Applicant: Honeywell Safety Products USA, Inc., Charlotte, NC 28202 (US)
(72) Inventor: WANG, Yuyan, Charlotte, 28202 (US); LIN, Ling, Charlotte, 28202 (US); ZHOU, Peng, Charlotte, 28202 (US); GU, Zheng, Charlotte, 28202 (US); ZHOU, Tianqi, Charlotte, 28202 (US)
(74) Representative: Haseltine Lake Kempner LLP

(57) **Abstract**

Various devices, assemblies, components, systems, and methods are provided relating to a waist padding assembly. An example assembly may include a center portion and two side portions. The center portion may comprise a porous ethylene-vinyl acetate (EVA) material and configured to contact a back of a user. The center portion may further comprise a warp mesh material and a 3D spacer fabric. The EVA material may comprise a plurality of holes to assist with the breathability of a waist padding assembly. The two side portions may be configured to secure to respective opposite sides of the center portion. The two side portions may comprise a polyurethane foam material configured to contact the flanks of the user.

## Description

### TECHNICAL FIELD

The present disclosure relates to a waist padding assembly usable with a harness, which may be capable of providing an operator with a breathable, ergonomic padding. Various components, materials, assemblies, devices, and methods are also provided.

### BACKGROUND

Harnesses may be used to protect operators in various fall prevention situations. A harness may include limited padding to support the operator. Traditional waist paddings are made of a single material due to which the paddings do not distribute the force effectively. Such traditional harnesses do not provide sufficient breathing space. Through applied effort, ingenuity, and innovation, Applicant has solved problems relating to waist padding assemblies and associated harnesses by developing solutions embodied in the present disclosure, which are described in detail below.

### BRIEF SUMMARY

Various embodiments of the present disclosure include padding, assemblies, harnesses, and corresponding systems, devices, components, and methods relate to waist padding assembly.

Various embodiment of the present disclosure may include a waist padding assembly. The waist padding assembly may include a center portion. The center portion may comprise a porous ethylene-vinyl acetate (EVA) material. The center portion may be configured to contact a back of a user. The waist padding assembly may further include two side portions comprising polyurethan foam. The two side portions may respectively connect to opposite sides of the center portion. The two side portions may be configured to contact respective flanks of the user.

In various embodiments, the center portion may comprise a first side and a second side, wherein the first side may be configured to contact the back of the user. In some embodiments, the center portion may further comprise a warp mesh material. The warp mesh material may be disposed along the second side of the center portion. In some embodiments, the warp mesh material may be configured to cover a portion of the porous EVA material of the center portion. In some embodiments, the center portion may further a 3D spacer fabric, wherein the 3D spacer fabric may be disposed along the first side of the center portion, such that, the 3D spacer fabric is configured to contact the back of a user. The 3D spacer fabric may be configured to cover a second portion of the porous EVA material of the center portion. In some embodiments, the 3D spacer fabric may be disposed opposite a warp mesh material relative to the porous EVA material, wherein the 3D spacer fabric and warp mesh material may at least in part be parallel to each other.

In some embodiments, the porous EVA material of the center portion may further comprise a plurality of holes. The plurality of hole may be configured to align with each other and may be configured to be oriented towards a back of a user, such that the plurality of holes may be configured to assist in the breathability of the center portion. In some embodiments, the plurality of holes may be configured to a length of the center portion.

In some embodiments, a waist padding assembly may further comprise one or more edge covers. The one or more edge covers may be configured to be disposed circumferentially around an edge of the center portion and the two side portions.

Various embodiments of the present disclosure may include a harness assembly. The harness assembly may include a waist padding assembly. In some embodiments, a waist padding assembly may comprise waist padding assembly may include a center portion. The center portion may comprise a porous ethylene-vinyl acetate (EVA) material. The center portion may be configured to contact a back of a user. The waist padding assembly may further include two side portions comprising polyurethan foam. The two side portions may respectively connect to opposite sides of the center portion. The two side portions may be configured to contact respective flanks of the user. In some embodiments, the harness assembly may include a backplate connected to the waist padding assembly at or proximate the center portion. In some embodiments, the harness assembly may include at least one strap. The at least one strap may be configured to secure the waist padding assembly to the user.

In some embodiments, the center portion of a harness assembly may comprise a first side and a second side, wherein the first side may be configured to contact the back of the user. In some embodiments, the center portion may further comprise a warp mesh material. The warp mesh material may be disposed along the second side of the center portion.

In some embodiments, the center portion of a harness assembly may further comprise a 3D spacer fabric. The 3D spacer fabric may be disposed along the first side of the center portion, such that, the 3D spacer fabric contacts the back of a user. The 3D spacer fabric may be configured to cover a second portion of the porous EVA material of the center portion. In some embodiments, the porous EVA material of a harness assembly may further comprise a plurality of holes. The plurality of holes may align with each other and may be configured to be oriented towards a back of a user, such that the plurality of holes may be configured to assist in the breathability of the center portion. In some embodiments, the plurality of holes may be configured to be perpendicular to a length of the center portion.

In some embodiments, a harness assembly may further comprise one or more edge covers. The one or more edges covers may be circumferentially disposed around an edge of the center portion and the two sides. In some embodiments, the harness assembly may further comprise a belt. The belt may be configured to be disposed along a length of the waist padding assembly. In some embodiments, the belt may further comprise at least two attachment rings. The at least two attachment rings may be disposed on opposite ends if the belt adjacent to the respective two side portions of the waist padding assembly.

In some embodiments, a harness assembly may further comprise a retainer. The retainer may secure to a portion of the waist padding assembly and may be configured to define a passage. A belt may be configured to pass through the passage.

Various embodiments may include a method of manufacturing a waist padding assembly. The waist padding assembly may comprise a center portion that may comprise a porous ethylene-vinyl acetate (EVA) material. The center portion may be configured to contact a back of a user. Two side portions may comprise polyurethan foam. The two side portions may be configured to be respectively connected to opposing sides of the center portion and may be configured to contact respective flanks of the user. The method may include attaching the two side portions to either of the opposing sides of the center portion.

The above summary is provided merely for purposes of summarizing some example embodiments to provide a basic understanding of some aspects of the present disclosure. Accordingly, it will be appreciated that the above-described embodiments are merely examples and should not be construed to narrow the scope or spirit of the present disclosure in any way. It will be appreciated that the scope of the present disclosure encompasses may potential embodiments in addition to those here summarized, some of which will be further described below. Other features, aspects, and advantages of the subject matter will become apparent from the description, the drawings, and the claims.

### BRIEF DESCRIPTION OF THE DRAWINGS

The following drawings are illustrations of a particular embodiments of the present disclosure and therefor do not limit the scope or spirit of the present disclosure. The drawings are not necessarily drawn to scale, nor are they necessarily are intended for use in conjunction with the explanation in the following detailed description.
Fig. 1 illustrates a front view of an exemplary waist padding assembly in accordance with various embodiments of the present disclosure;
Fig. 2 illustrates a top view of an exemplary waist padding assembly with a backplate in accordance with various embodiments of the present disclosure;
Fig. 3 illustrates a side view of an exemplary waist padding assembly with a backplate in accordance with various embodiments of the present disclosure;
Fig. 4 illustrates a back perspective view of an exemplary waist padding assembly with a backplate in accordance with various embodiments of the present disclosure;
Fig. 5 illustrates a front perspective view of a waist padding assembly in accordance with various embodiments of the present disclosure;
Fig. 6 illustrates a front perspective exploded view of a waist padding assembly with a backplate in accordance with various embodiments of the present disclosure;
Fig. 7 illustrates another front perspective exploded view of a waist padding assembly with a backplate in accordance with various embodiments of the present disclosure;
Fig. 8 illustrates a cross-sectional view of a center portion of a waist padding assembly in accordance with various embodiments of the present disclosure;
Fig. 9 illustrates an exploded view of a plurality of layers of the center portion in accordance with various embodiments of the present disclosure;
Fig. 10 illustrates a back perspective view of a harness assembly in accordance with various embodiments of the present disclosure; and
Fig. 11 illustrates a side perspective view of a harness assembly secured to a operator in accordance with various embodiments of the present disclosure.

### DETAILED DESCRIPTION

Some embodiments of the present disclosure will be described in a more detailed manner hereinafter with reference to the accompanying drawings, in which some, embodiments of the invention are shown. Reference numbers refer to elements throughout the drawings. Multiple embodiments of the current invention may be embodied in different forms and should not be limited to the embodiments set forth herein. Rather, these embodiments are provided so that this disclosure will satisfy applicable legal requirements.

As used herein, terms such as "front," "rear," "top," etc. are used for explanatory purposes in the examples provided below to describe the relative positions of certain components or portions of components relative to other components or portions of components. As used herein, the term "or" is used in both the alternative and conjunctive sense, unless otherwise indicated. The term "along," and similarly utilized terms, means near or on, but necessarily requiring directly on an edge or other referenced location. The terms "approximately," "generally," and "substantially" refer to within manufacturing and/or engineering design tolerance for the corresponding materials and/or elements unless otherwise indicated. The use of such term is inclusive of and is intended to allow independent claiming of specific values listed. Thus, use of any such aforementioned terms, or similarly interchangeable terms, should not be taken to limit the spirit and scope of embodiments of the present invention. As used in the specification and the appended claims. The singular form of "a," "an," and "the" include plural references unless otherwise stated. The terms "includes" and/or "including," when used in the specification, specify the presence of stated features, elements, and/or components, and/or groups thereof.

As used herein, the phrases "in one embodiment," "according to one embodiment," "in some embodiments," and the like generally refer to the fact that the particular feature, structure, or characteristic following the phrase may be included in at least one embodiment of the present disclosure. Thus, the particular feature, structure, or characteristic may be included in more than one embodiment of the present disclosure such that these phrases do not necessarily refer to the same embodiment. As used herein, the terms "example," "exemplary," and the like are used to "serving as an example, instance, or illustration." Any implementation, aspect, or design described herein as "example" or "exemplary" is not necessarily to be construed as preferred or advantageous over other implementations, aspects, or designs. Rather, use of the terms "example," "exemplary," and the like are intended to present concepts in a concrete fashion.

The figures are provided to illustrate some examples of the invention described. The figures are not to limit the scope of the present embodiment of the invention or the appended claims. Aspects of the example embodiments are described below with reference to example applications for illustration. It should be understood that specific details, relationships, and methods are set forth to provide a full understanding of the example embodiments. One of ordinary skill in the art recognize the example embodiment can be practice without one or more specific details and/or with other methods.

The present disclosure relates to a waist padding assembly comprising multiple portions made of various materials at various locations of the padding assembly to allow for improved force distribution, support, breathability, and ergonomics for an operator (also referred to as a "user"). Various components, materials, assemblies, devices, and methods are also provided. In some example embodiments, the waist padding assembly herein may be used to contact the back of a user at or proximate the user's waistline as part of a harness assembly. Various harnesses and padding arrangements for harnesses typically lack the ability to provide equal distribution of forces applied to the user's waist, lack breathability, provide poor ergonomic support (e.g., create pressure points), and/or are uncomfortable to wear. For example, the uneven distribution of force can cause discomfort to operators who may be required to wear the harness for extended periods of times while performing operations of daily work. Embodiments of the present disclosure solve one or more of the aforementioned problems.

In some embodiments, a waist padding assembly may be provided with a center portion and two side portions. The two side portions may be disposed on opposite sides of the center portion. While being worn, the center portion may be configured to substantially align with and at least partially contact the user's back and the two side portions may be configured to substantially align with and at least partially contact the user's respective left and right flanks (e.g., along the user's waistline). The center portion may be made of one or more materials, while the two side portions may include at least one different material. In some embodiments, the center portion may comprise a porous ethylene-vinyl acetate (EVA) material. The EVA material may be configured to be breathable while distributing forces along the user's back. For example, in some embodiments, the EVA material may be porous, including ventilation holes defined therein to allow cooling of the user's back. Each of the two side portions comprise a different material configured to provide lateral support to the operator. For example, the side portions may comprise a polyurethane (PU) foam. In some embodiments, the side portions may be sewn, adhered, and/or otherwise attached to the center portion.

In operation, during extended use, the center portion may experience constant or nearly constant pressure in the form of the operator's weight resting against the center portion. In such embodiments, the center portion may be made of a higher density and higher resilience material (e.g., EVA) than the side portions to offer strong support for the lumbar during use. In some embodiments, the two side portions may be configured to distribute wight and/or pressure from an operator when the operator applies additional pressure to the side portions during periodic movements (e.g., leaning, and/or the like). In some embodiments, the two side portions may comprise a PU foam, such as an integral skin foam (ISF). In some embodiments, ISF foam in the side portions may be configured to distribute pressure in the vertical direction. In various embodiments, example ISF side portions may be configured to recover to an initial state without plastic deformation (e.g., a compression set test result of 0 deformation after 24 hours compression). In some embodiments, the EVA material of the center portion may comprise a more rigid material than the ISF, wherein the EVA material of the center portion may be configured to provide more support to the lumbar of a user. In some embodiments, the EVA material of the center portion may be configured to distribute the pressure in a horizontal direction. In some embodiments, the EVA may be a high-density EVA (e.g., 65kg/m³) with a higher hardness than the PU side portions (e.g., HC70).

In some embodiments, a back plate may provide further support to the center portion. The back plate may be made of, for example, thermoplastic polyurethane (TPU) (e.g., nylon) and may provide at least some additional rigidity to the EVA material. In some embodiments, the center portion may additionally include a 3D spacer fabric disposed along a first side of the center portion. The 3D spacer fabric may be configured to add breathability and comfort for the user. The first side of the center portion may be configured to contact the user's back. In some embodiments, the center portion may additionally include a warp mesh material disposed along a second side of the center portion, which may facilitate breathability while covering the EVA material. In some embodiments, the side portions may be attached directly to the EVA material.

In various embodiments, the waist padding assembly may include one or more retainers, which may define a passage to receive a belt configured to wrap around the user's waist, with the EVA material and PU material of the respective center and side portions being configured to be disposed between the user's waist and the belt. The belt may comprise one or more attachment rings configured to engage a rope, wire, clip, carabiner, pulley, or other retaining device through which at least some of the weight of the user is supported and force thereby applied to the user's waist while being cushioned and supported by the waist padding assembly.

As described herein, various embodiments of the present disclosure may include a waist padding assembly that utilizes a porous material and two side portions to provide an operator with improved force distribution, increased breathability, and increased ergonomics to ensure better wearability and long-term comfort.

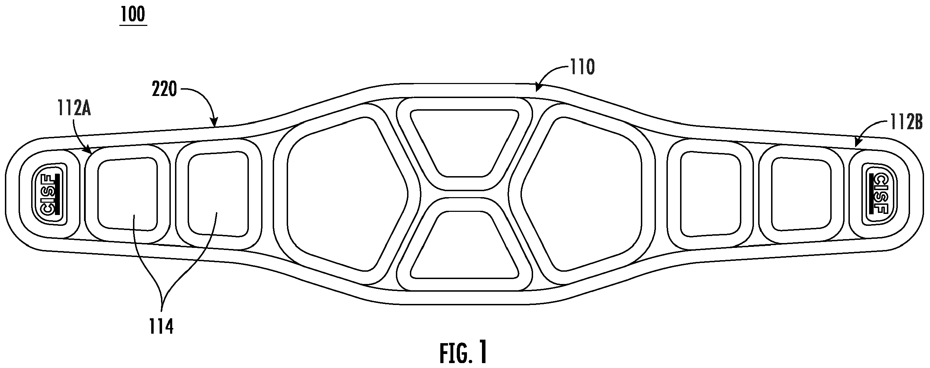

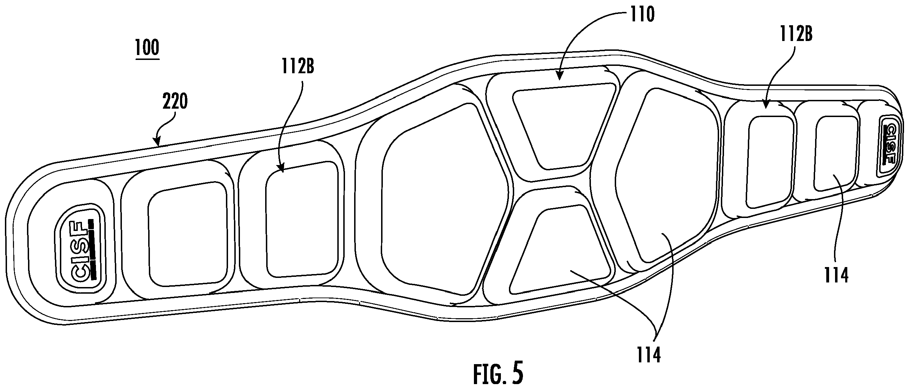

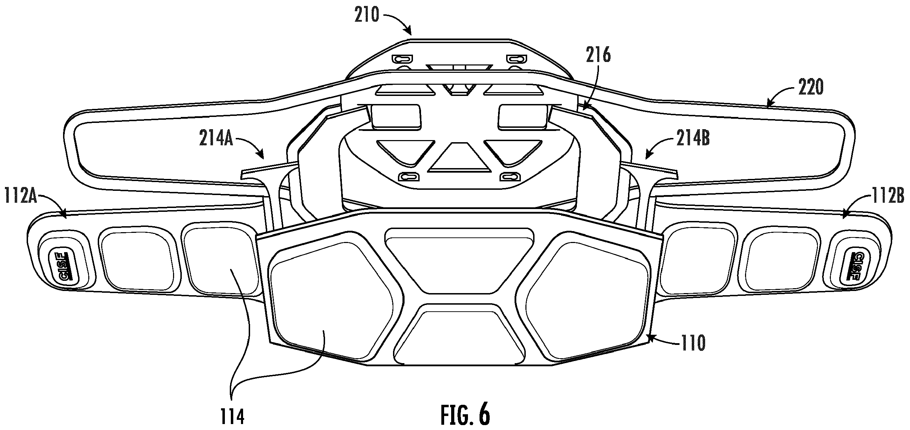

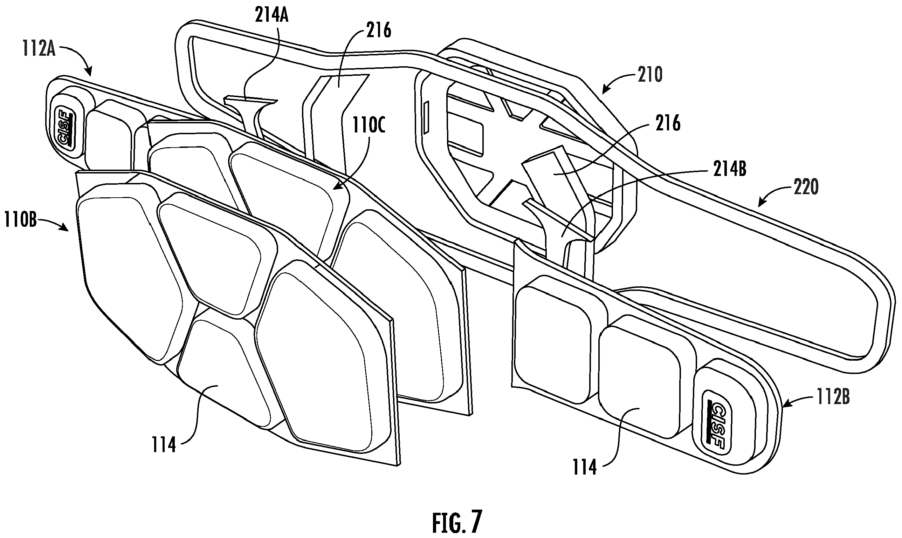

Figs. 1-11 depict exemplary views of an example waist padding assemblies and a harness assemblies and portions thereof in accordance with various embodiments of the present disclosure. Figs. 1 and 5 depict a front view and a front perspective view of a waist padding assembly 100. The waist padding assembly 100 may comprise a center portion 110 and/or two side portions 112A, 112B (collectively "112"). In the depicted embodiment, the side portions 112A, 112B (e.g., a first side portion 112A and a second side portion 112B) are shown on opposite sides of the center portion 110. Figs. 1-5 depict the center portion 110 and side portions 112A, 112B affixed as a single padding assembly, while Fig. 6 illustrates and exploded view prior to such assembly. Each of the side portions 112A, 112B may be secured to the center portion 110, for example, via affixation such as sewing, adhesive, and/or other means of attachment.

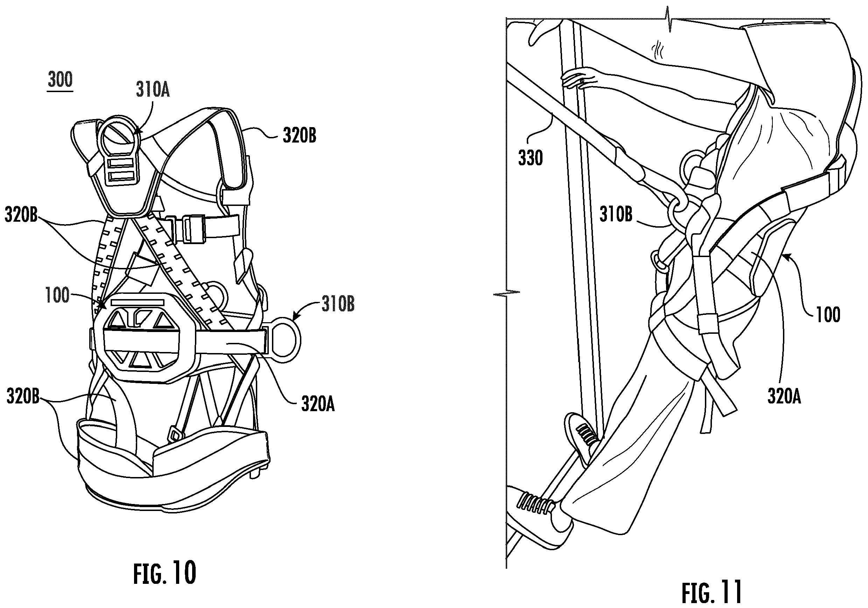

In various embodiments, two side portions 112 may comprise at least one different material than the center portion. In some embodiments, the side portion 112 may comprise a polyurethane (PU) foam material (e.g., HC28 hardness). The polyurethane material may be configured to help distribute peak pressure forces endured by an operator and to distribute the pressure across an area of the operator's flanks to reduce the maximum pressure on any one point or sub-area of the flanks. For example, in operation, the harness assembly (e.g., harness assembly 300 shown in Figs. 10-11) may include a belt (e.g., belt 320B shown in Figs. 10-11) that wraps around the user's back along or proximate the waistline. In some instances, the belt 320B may apply pressure to the operator's flanks due to the pinching action of the opposing sides of the belt coming together as the operator's body weight applies force to the belt. For example, as tension is applied to the illustrated O-rings 310B by the retainer device 330 illustrated in Fig. 11, the opposite sides of the belt may be urged towards each other, sandwiching the user therebetween. Thus, the cushioning ability and rigidity of the side portions may resist this force from the belt and distribute the force more evenly to reduce the peak pressure on the wearer and the peak force at any specific point relative to having a weaker (or no) side portion. In some embodiments, PU side portions 112 may provide improved performance under compression to consistently distribute the stress across the user's waistline. In various embodiments, the two side portions 112 may secure to the center portion 100 via any manner of attachment (e.g., sewing, adhesion, etc.). In some embodiments, the first side of the center portion 110 and/or the side portions 112 may be contoured, such as with the raised plateaus 114 illustrated in Figs. 1-3, 5-7. In some embodiments, the EVA material may be contoured and/or a surface layer of 3D spacer fabric may be contoured.

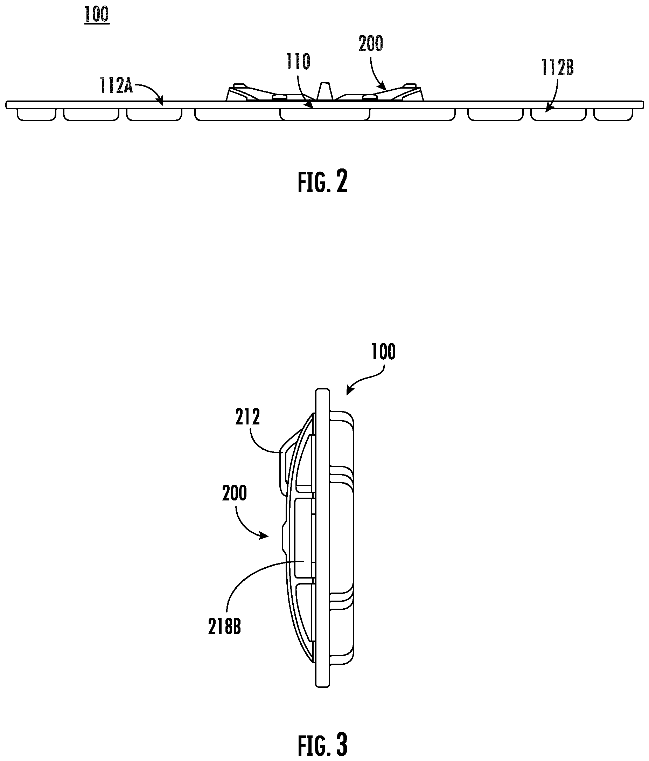

With reference to Figs. 2-4 and 6-7, a back plate 200 may be attached, directly or indirectly, to the center portion 110 of the waist harness padding 100. In some example embodiments, the waist padding assembly 100 may engage directly with the backplate 200. In various embodiments, the backplate 200 may secure to the waist padding assembly 100 via any manner of attachment (e.g., sewing, adhesion, etc.). In various embodiments, the backplate 200 may secure to the waist padding assembly 100 at the center portion 110. For example, the back plate 200 may be the same shape or substantially the same shape as the center portion 110 and may be configured to align with the footprint of the center portion 110 to provide additional rigidity to the center portion. In other embodiments, the backplate 200 may secure to the waist padding assembly 100 proximate to the center portion 110. The backplate may, for example, be made of a rigid material, such as thermoplastic polyurethane (TPU) and configured to limit or prevent flexion of the center portion caused by the belt (e.g., belt 320A shown in Figs. 10-11) being pulled from either end.

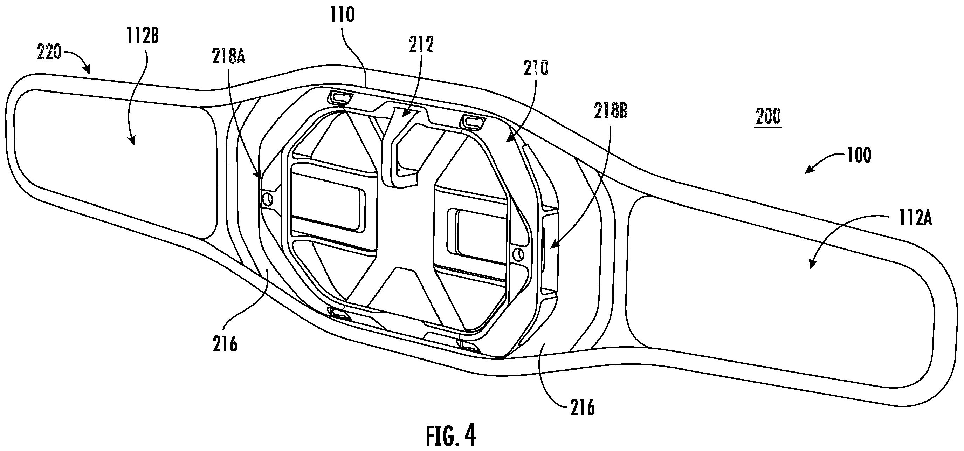

With reference to Fig. 4, a rear perspective view of an example backplate 200 is depicted attached with a waist padding assembly 100 in accordance with various embodiments of the present disclosure. In various embodiments, the backplate 200 may comprise a shell 210 forming a support structure that provides rigidity to at least part of the center portion 110 of the waist padding assembly 100. The shell 210 may define one or more hook elements 212 and/or one or more retainers 218A, 218B (collectively "218"). For example, the hook element(s) 212 may form an attachment point for one or more retainers (e.g., carabiners) and/or any other component or accessory. In some embodiments, the retainers 218 may form a passage through which the belt (e.g., belt 320A shown in Figs. 10-11) is configured to pass. In some embodiments, the belt may pass around an exterior of the backplate opposite the center portion 110. The retainers 218 may be configured to vertically align the belt with the waist padding assembly and allow the backplate 200 to absorb some of the force from the belt while protecting and distributing the force received by the user. In various embodiments, the backplate shell 210 may comprise one or more cutouts within the shell. The one or more cutouts of the backplate shell 210 may assist in the increase airflow to an operator and/or the breathability of the center portion 110.

In various embodiments, the backplate 200 may comprise a rigid plastic material (e.g., TPU such as nylon, etc.) configured to provide additional support to the lower back of an operator and/or the lower portion of an operator's waist. In various embodiments, the shell 210 of the backplate may be configured to engage with a second side of the waist padding assembly 100. In some embodiments, the one or more hook elements 212 and/or the one or more retainers 218 may integral with the shell 210 of the backplate. The one or more retainers 218A, 218B may be disposed on opposite sides of the backplate shell 210, such that a first retainer 218A is on a first side and a second retainer 218B is on a second side.

With even further reference to Fig. 4, in various embodiments, the waist padding assembly 100 may comprise one or more edge covers 220 disposed around the center portion 110 and/or side portions 112. In the depicted embodiment, an edge cover 220 is disposed circumferentially around the edge of the connected center portion 110 and side portions 112. In various embodiments, the one or more edge covers 220 may be configured to encompass the entire outer edge of the first side portion 112A, the second side portion 112B, and the center portion 110. The one or more edge covers 220 may be affixed to the outer edge of the waist padding assembly 100 in a plurality of manners (e.g., sewing, adhesion, etc.). In some embodiments, the edge cover(s) 220 may comprise polyurethane (PU). In some embodiments, the edge cover(s) 220 may comprise tape configured to attach to the edge of the waist padding assembly 100. In some embodiments the edge cover(s) 220 may comprise a lining tape coated with PU.

With reference to Figs. 6-7, exploded views of the waist padding assembly 100 and backplate 200 are depicted in accordance with various embodiments of the present disclosure. In various embodiments, the waist padding assembly 100 and the backplate 200 may be configured to secure together, and the backplate 200 may secure to the second side of the waist padding assembly. In some embodiments, the assembly may further comprise one or more retainers 214A, 214B (collectively "214") that are separate from the backplate 200 and may additionally or alternatively define passages for the belt. For example, the illustrated retainers 214 are separate fabric straps configured to extend vertically between a top and bottom of the waist padding assembly 100 (e.g., with the edge cover 220 extending thereover). In some embodiments, the one or more separate retainers 214 may be configured to affix to opposite sides of the center portion 110. In some embodiments, the one or more separate retainers 214 may be disposed at the respective joints of the side portions 112 and center portion 110 of the waist padding assembly 100. The one or more separate retainers 214 may comprise a leather (e.g., nubuck) or leather-like material (e.g., synthetic leather, such as PU leather or polyvinyl chloride (PVC) leather). In some embodiments, the one or more separate retainers 214 may be included in addition to or instead of the retainers 218 of the backplate 200. In some embodiments, the one or more separate retainers 214 may be configured to receive a belt (e.g., belt 320A shown in Figs. 10-11) therethrough.

In various embodiments, the assembly may comprise at least one reflective section 216 visible from a rear of the harness assembly. The at least one reflective section 216 may be affixed to the center portion 110 and/or side portion(s) 112. In the embodiment depicted in FIG. 4, two reflective sections 216 are shown attached to the center portion 110 at either side of the shell 210 of the backplate 200, with the backplate supporting only a center section of the center portion. The retainer(s) 214 and/or reflective section(s) 216 may be integrated into the assembly via any attachment (e.g., sewing, adhesion, etc.).

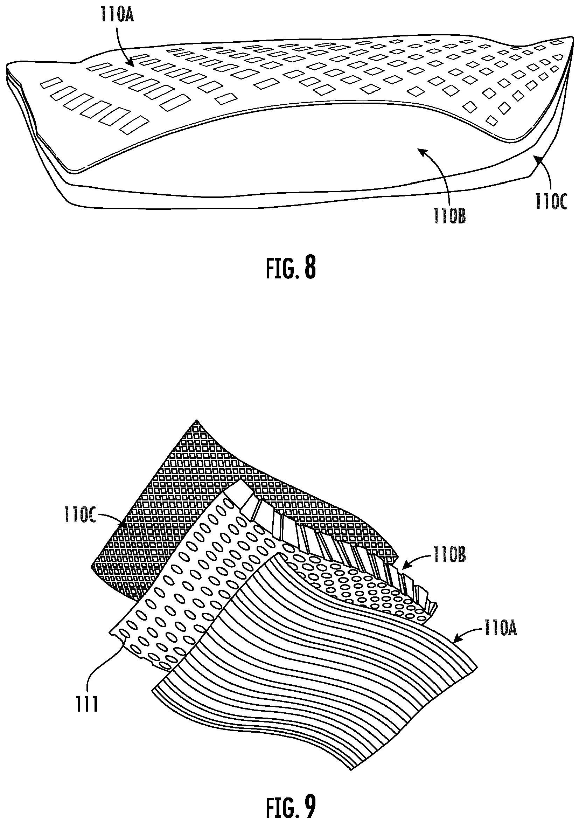

With reference to Figs. 8-9, a cross-sectional view and exploded perspective view of the material of the center portion 110 are depicted in accordance with various embodiments of the present disclosure. In various embodiments, the center portion 110 may comprise two or more different materials. In some embodiments, at least one of the materials may be a porous ethylene-vinyl acetate (EVA) material with at least one layer at the front (e.g., first side, facing the user) and/or at the rear (e.g., the second side, facing away from the user towards the backplate). In the embodiment depicted in Figs. 8-9, the center portion 110 comprises at least three layers (e.g., front layer 110A, middle layer 110B, and rear layer 110C). In various embodiments, the front layer 110A may comprise a 3D spacer fabric material, the middle layer 110B may comprise the porous EVA material, and/or the rear layer 110C may comprise the warp mesh material. The 3D spacer fabric may be disposed along the first side of the center portion 110 and configured to engage with a first side of the middle layer 110B of the center portion 110 (e.g., the porous EVA material). The warp mesh material may be disposed along the second side of the center portion 110 may configured to engage with a second side of the middle layer 110B of the center portion 110 (e.g., the porous EVA material). In various embodiments, the front layer 110A (e.g., the 3D spacer fabric) and the rear layer 110C (e.g., the warp mesh material) may be disposed linearly opposite of each other on opposing sides of the middle layer 110B. The warp mesh material layer 110C and the 3D spacer fabric layer 110A may be at least in parallel to each other relative to a length of the waist padding assembly 100. In some embodiments, the side portions 112 may comprise only PU foam.

With further reference to Fig. 8, in various embodiments, a first side of the center portion (e.g., first layer 110A) may be configured to contact the back of the harness operator. The first layer 110A of the center portion 100 may provide at least some cushion and breathability upstream of the middle layer 110B. The 3D spacer fabric material may be configured to absorb moisture created from the contact between the center portion and the back of the operator while creating voids (e.g., via the 3D shape of the fabric and/or the pores in the fabric) for ventilation and evaporation of the moisture. The 3D spacer fabric material may further allow for increase air flow to and through the middle layer 110B.

3D spacer fabric may comprise multiple layers itself. The 3D spacer fabric may include two surface layers on opposing sides with a "spacer" layer between the two surface layers. In some embodiments, the surface layers may be a mesh construction to allow airflow therethrough. The spacer layer may comprise vertical filler strands that define a thickness (e.g., variable or constant thickness along the fabric) of the 3D spacer fabric. In some embodiments, the 3D spacer fabric thickness may be adjusted to define a pattern, such as the waffle pattern of the front layer shown in Fig. 8 or the linear pattern shown in Fig. 9. The 3D spacer layer may allow ventilation with its porous design and/or with its patterned surface thickness to facilitate airflow between the surface layers and parallel to the surface layers. The warp mesh fabric may be warp knit into a mesh to provide strength and breathability.

With further reference to Fig. 9, in various embodiments, the porous EVA material may comprise a plurality of holes 111. In some embodiments, the plurality of holes 111 may be arranged in a pattern throughout the EVA material to control the porosity of and thus the airflow through the depicted middle layer 110B. The plurality of holes may be aligned with each other (e.g., aligned along a same or substantially the same direction relative to a flat configuration of the EVA material) and configured to be oriented towards a back of a user (e.g., from front-to-rear in the depicted orientation), such that the plurality of holes are configured to assist in the breathability of the center portion 110. In the depicted embodiments, the plurality of holes 111 are oriented perpendicular to a length of the waist padding assembly 100. While the length of the waist padding assembly may not necessarily be linear depending on its orientation, the plurality of holes 111 may be oriented perpendicular to the section of length immediately adjacent the holes. In various embodiments, the plurality of holes 111 may be configured to extend fully through the middle layer 110B from the first side of the EVA material through the second side of the EVA material to allow air to flow therethrough. In various embodiments, the plurality of holes 111 may assist with airflow through the center portion 110, wherein the increased airflow may create better breathability of the waist padding assembly 100. In one or more embodiments, the rear layer 110C (e.g., the warp mesh material) disposed along the second side of the center portion 110 may facilitate the breathability of the waist padding assembly may allowing airflow between an exterior environment and the middle layer 110B and front layer 110A. In some embodiments the rear layer 110C may be configured to connect, directly or indirectly, with the backplate 200, retainer(s) 214, and/or reflective section(s) 216.

With further reference to Fig. 9, in various embodiments, the rear layer 110C (e.g., the warp mesh material) may be configured to cover a first portion of the middle layer 110B (e.g., the EVA material). The rear layer 110C may be affixed to a second (e.g., rear) side of the middle layer through various means of attachment (e.g., sewn, adhered, etc.). In various embodiments, at least one side portions may be configured attached (e.g., sewn, adhered, etc.) to the opposite sides of the middle layer 110B before the attachment of a front layer 110A. The front layer 110A (e.g., 3D spacer fabric) may be configured to be attached to a first (e.g., front) side of the middle layer 110B, wherein the front layer 110A may be affixed to the middle later 110B after the attachment of one or more side portions. The rear layer may be affixed to the one or more side portions and/or middle layer through various means of attachment (e.g., sewn, adhered, etc.). In various embodiments, the rear layer 110C may be configured to engage with a backplate of the harness assembly. In one example embodiment, the waist padding assembly may be assembled in the following order: (1) rear layer (e.g., warp mesh) and middle layer (e.g., EVA) attached together; (2) side portions sewn onto either side of the result of step (1); and (3) first layer (e.g., 3D spacer fabric) sewn to the result of step (2).

With reference to Figs. 10-11, in various embodiments the waist padding assembly may be configured to be a portion of a harness assembly in accordance with various embodiments of the present disclosure. In one or more embodiments, the waist padding assembly 100 may be configured to be a portion of a harness assembly 300. The harness assembly 300 may be worn by an operator, for example, to protect the operator from falls (e.g., in a utility, industrial, recreational, or other elevated operating situation). The harness assembly 300 may include a plurality of straps 320A, 320B for engaging and/or supporting the operator, in accordance with known harness configurations but for the presently improved assemblies.

The plurality of straps may include a belt 320A configured to support at least a portion of the operator's weight as part of the harness assembly. In the embodiment depicted in Figs. 10-11, the belt 320A is connected to an attachment ring 310B at each respective end. The attachment rings 310B may be configured to engage a retainer device 330 (e.g., a rope, wire, clip, carabiner, pulley, fall prevention device, or other retaining device) to apply a force to the belt via the attachment rings. The force applied to the belt thereby applies pressure to the user via the belt acting on the waist padding assembly 100 and backplate 200 as described herein. The belt 320 pass through one or more retainers (e.g., retainers 218 on the backplate shell 210 and/or separate retainers 214) to locate the belt against the waist padding assembly 100 and/or backplate 200 and direct the force of the belt therethrough to the user's waistline. The materials and structure of the waist padding assembly 100 and/or backplate 200 described herein may thereby improve force distribution, support, breathability, and ergonomics for the user to allow the user to comfortably work for extended periods. In some embodiments, the overall size (e.g., height and/or length) of the waist padding assembly may be adjusted to suit the size of the harness and/or operator.

Many modifications and other embodiments of the disclosure set forth herein will come to mind to one skilled in the art to which this disclosure pertains having the benefit of the teachings presented in the foregoing descriptions and the associated drawings. Therefore, it is to be understood that the disclosure is not to be limited to the specific embodiments disclosed and that modifications and other embodiments are intended to be included within the scope of the appended claims. Although specific terms are employed herein, they are used in a generic and descriptive sense only and not for purposes of limitation.

## Claims

1. A waist padding assembly comprising:
a center portion comprising a porous ethylene-vinyl acetate (EVA) material, the center portion configured to contact a back of a user; and
two side portions comprising polyurethane foam, the two side portions respectively connected to opposing sides of the center portion and configured to contact respective flanks of the user.

2. The waist padding assembly of claim 1, wherein the center portion comprises a first side and a second side, wherein the first side is configured to contact the back of the user.

3. The waist padding assembly of claim 2, wherein the center portion further comprises:
a warp mesh material, wherein the warp mesh material is disposed along the second side of the center portion and is configured to cover a portion of the porous EVA material of the center portion.

4. The waist padding assembly of claim 2, wherein the center portion further comprises:
a 3D spacer fabric, wherein the 3D spacer fabric is disposed along the first side of the center portion such that the 3D spacer fabric is configured to contact the back of the user, and the 3D spacer fabric is configured to cover a second portion of the porous EVA material of the center portion, and wherein the 3D spacer fabric is disposed opposite a warp mesh material relative to the porous EVA material, wherein the 3D spacer fabric and warp mesh material are at least in part parallel to each other.

5. The waist padding assembly of claim 1, wherein the porous EVA material of the center portion further comprises:
a plurality of holes, wherein the plurality of holes are aligned with each other and configured to be oriented towards a back of a user, such that the plurality of holes are configured to assist in the breathability of the center portion, and wherein the plurality of holes are configured to be perpendicular to a length of the center portion.

6. The waist padding assembly of claim 1, further comprises:
one or more edge covers circumferentially disposed around an edge of the center portion and the two side portions.

7. A harness assembly comprising:
a waist padding assembly comprising:
a center portion comprising a porous ethylene-vinyl acetate (EVA) material, the center portion configured to contact a back of a user; and
two side portions comprising polyurethane foam, the two side portions respectively connected to opposing sides of the center portion and configured to contact respective flanks of the user;
a backplate connected to the waist padding assembly at or proximate the center portion; and
at least one strap configured to secure the waist padding assembly to the user.

8. The harness assembly of claim 7, wherein the center portion comprises a first side and a second side, wherein the first side is configured to contact the back of the user.

9. The harness assembly of claim 8, wherein the center portion further comprises:
a warp mesh material, wherein the warp mesh material is disposed along the second side of the center portion and is configured to cover a portion of the porous EVA material of the center portion.

10. The harness assembly of claim 8, wherein the center portion further comprises:
a 3D spacer fabric, wherein the 3D spacer fabric is disposed along the first side of the center portion such that the 3D spacer fabric is configured to contact the back of the user, and the 3D spacer fabric is configured to cover a second portion of the porous EVA material of the center portion, and wherein the 3D spacer fabric is disposed opposite a warp mesh material relative to the porous EVA material, wherein the 3D spacer fabric and warp mesh material are at least in part parallel to each other

11. The harness assembly of claim 7, wherein the porous EVA material of the center portion further comprises:
a plurality of holes, wherein the plurality of holes are aligned with each other and configured to be oriented towards a back of a user, such that the plurality of holes are configured to assist in the breathability of the center portion, and wherein the plurality of holes are configured to be perpendicular to a length of the center portion.

12. The harness assembly of claim 7, further comprises:
one or more edge covers, circumferentially disposed around an edge of the center portion and the two side portions; and
a belt, wherein the belt is configured to be disposed along a length of the waist padding assembly.

13. The harness assembly of claim 13, wherein the belt further comprises:
at least two attachment rings, wherein the at least two attachment rings are disposed on opposite ends of the belt adjacent to the respective two side portions of the waist padding assembly.

14. The harness assembly of claim 14, the harness assembly further comprises:
a retainer, wherein the retainer is secured to a portion of the waist padding assembly and configured to define a passage, and wherein the belt is configured to pass through the passage.

15. A method of manufacturing a waist padding assembly, the waist padding assembly comprising a center portion comprising a porous ethylene-vinyl acetate (EVA) material, the center portion configured to contact a back of a user; and two side portions comprising polyurethane foam, the two side portions configured to be respectively connected to opposing sides of the center portion and configured to contact respective flanks of the user, the method comprising:
attaching the two side portions to either of the opposing sides of the center portion.
